# EUROPÄISCHE PATENTSCHRIFT

(11) **EP 1 428 501 B1**
(45) Veröffentlichungstag und Bekanntmachung des Hinweises auf die Patenterteilung: **14.07.2010**
(21) Anmeldenummer: 02028212.5
(22) Anmeldetag: 14.12.2002
(51) Int. Cl.: A61K 8/04, A61K 8/81, A61Q 5/06

(54) **Aerosolschaumprodukt zur Haarbehandlung**
Aerosol foam product for treating hair
Mousse aérosol pour traitement capillaire

(43) Veröffentlichungstag der Anmeldung: 16.06.2004
(73) Patentinhaber: The Procter and Gamble Company, Cincinnati, OH 45202 (US)
(72) Erfinder: Wendel, Harald, 64372 Ober-Ramstadt (DE); Birkel, Susanne, Dr., 64285 Darmstadt (DE); Franzke, Michael, Dr., 64380 Rossdorf (DE); Stähle, Liane, 64372 Ober-Ramstadt (DE)
(74) Vertreter: Marollé, Patrick Pierre Pascal

(56) Entgegenhaltungen:
- EP-A- 0 955 033
- DE-A- 10 101 382
- US-A1- 2001 022 967
- NATIONAL STARCH & CHEMICAL * PERSONAL CARE: "Modify rheology: STRUCTURE 2001 / STRUCTURE 3001 [Technical bulletin]" ONLINE, [Online] XP002209084 Gefunden im Internet: <URL:http://www.personalcarepolymers.com/S ite/List.asp?ID=Enhance.7>> [gefunden am 2002-08-08]

## Beschreibung

Gegenstand der Erfindung ist ein Aerosolschaumprodukt zur Haarbehandlung umfassend eine Zusammensetzung mit einem Gehalt an einem teilweise oder vollständig neutralisierten Itaconsäuremonoester/Acrylat Copolymer in einem geeigneten Lösungsmittel, abgefüllt mit einem Treibgas in einer druckfesten Verpackung mit einer Vorrichtung zum Verschäumen der Zusammensetzung.

Schäume zur Haarbehandlung sind bekannt und bestehen in der Regel aus haarfestigenden oder haarpflegenden Substanzen, Schaumbildnern sowie einer geeigneten, in der Regel wasserhaltigen Lösungsmittelbasis. Die Zusammensetzungen werden mittels eines Treibgases oder mittels einer mechanisch betriebenen Pumpschaumvorrichtung unmittelbar vor der Anwendung verschäumt. An die Qualität von derartigen Schaumprodukten werden verschiedene Anforderungen gestellt, die grob in zwei Gruppen aufgeteilt werden können. Die eine Gruppe von Qualitätsanforderungen betrifft die Schaumqualität, d.h. die Beschaffenheit des Schaumes. Hierzu gehören etwa die Feinoder Grobporigkeit, die Kompaktheit, die zeitliche Stabilität des Schaumes, die Einarbeitbarkeit und Verteilbarkeit auf dem Haar etc.. Die zweite Gruppe von Qualitätsanforderungen betrifft die Wirkungen, die der Schaum nach Einarbeitung in das Haar auf dem Haar hervorruft, d.h. die haarpflegenden Eigenschaften wie z.B. der Griff des Haares im feuchten und trockenen Zustand, die Kämmbarkeit, die Festigung, die Belastung des Haares, den Glanz des Haares etc.. Bei der Optimierung von Schaumprodukten besteht das Problem, dass durch Zusatz spezieller Wirkstoffe zwar eine Verbesserung einiger Qualitätsanforderungen, beispielsweise der Schaumqualität, erreicht werden kann, dies aber mit einer Verschlechterung anderer Qualitätsanforderungen, beispielsweise der haarpflegenden oder haarfestigenden Eigenschaften, erkauft wird.

Während der Einarbeitung in das Haar müssen die Schäume möglichst schnell und vollständig zerfallen. Herkömmliche Schäume sind daher relativ instabil, d.h. sie zerfallen auch ohne äußere Einwirkung innerhalb weniger Minuten. Diese Schäume fühlen sich wässrig an und ein kreativer Umgang mit diesen Schäumen ist nicht oder nur eingeschränkt möglich. Wünschenswert sind Schäume, die einerseits eine verbesserte Haptik und eine größere Stabilität aufweisen wenn sie nicht oder nur gering geschert werden und z.B. bei geringer Druckeinwirkung stabil formbar sind, die aber andererseits bei verstärkter Scherung bzw. höherer Druckeinwirkung während der Einarbeitung in das Haar dennoch leicht, schnell und rückstandsfrei zerfallen. Die größere Stabilität und die Formbarkeit bei geringen Druckeinwirkungen erlaubt einen kreativen Umgang mit den Schäumen. Außerdem sollen die Schäume gute Eigenschaften hinsichtlich Glanz der Haare, Lockendefinition, Haarfestigung und/oder Stabilität der Frisur aufweisen. Insbesondere der Glanz der Haare ist bei Anwendung herkömmlicher Aerosolhaarschäume häufig noch unbefriedigend. Es sind zwar eine Reihe von Additiven bekannt, welche in der Lage sind, den Haarglanz zu verbessern, z.B. hydrophobe Stoffe wie flüssige Paraffine, Isoparaffine, Silikonöle oder hydrophile Stoffe wie mehrwertige Alkohole, insbesondere Glycerin oder Propylenglykol. Die bekannten Glanzgeber wirken sich aber häufig nachteilig auf andere, erwünschte Eigenschaften eines Haarschaums aus. Sie können als Weichmacher für das verwendete festigende Polymer wirken und so die Festigungsleistung herabsetzen oder sie können als Entschäumer wirken und die Schaumqualität beeinträchtigen, oder sie sind in ihrer Haarglanz erzeugenden Wirkung noch nicht ausreichend.

Es wurde nun gefunden, dass Schäume zur Haarbehandlung mit besonderen haptischen Eigenschaften, hoher Stabilität, elastischen Eigenschaften, guter Einarbeitbarkeit und guten Anwendungseigenschaften auf dem Haar hergestellt werden können unter Verwendung von bestimmten Copolymeren in dem nachfolgend beschriebenen Schaumprodukt. Dabei wird unter einem Aerosolschaumprodukt ein Produkt verstanden, welches aus einer fluiden, verschäumbaren Zusammensetzung besteht, welche zusammen mit einem Treibmittel in einer druckdichten Verpackung abgefüllt ist, die Verpackung mit einer Vorrichtung zum Verschäumen (z.B. Schaumkopf) versehen ist und die Zusammensetzung unmittelbar vor der Anwendung in Form eines Schaumes ausgebracht werden kann.

Gegenstand der Erfindung ist ein Aerosolschaumprodukt zur Haarbehandlung, umfassend eine verschäumbare Zusammensetzung mit einem Gehalt an
(A) mindestens einem Copolymeren, welches gebildet ist aus mindestens einer ersten radikalisch polymerisierbaren Monomerart, ausgewählt aus Itaconsäuremonoestern der allgemeinen Formel CH₂=C(COOR¹)CH₂COOR² wobei einer der Substituenten R¹ und R² für Wasserstoff und der andere für die Gruppe -(CH₂CH₂O)ₓ-R³ steht, x eine Zahl zwischen 1 und 100 bedeutet und R³ eine Alkylgruppe mit 8 bis 30 C-Atomen bedeutet und mindestens einer zweiten radikalisch polymerisierbaren Monomerart, ausgewählt aus Acrylat-Monomeren
(B) mindestens einer Base zur teilweisen oder vollständigen Neutralisation des Copolymers (A) und
(C) einem geeigneten Lösungsmittelsystem,
wobei die Zusammensetzung zusammen mit mindestens einem Treibmittel in einer druckfesten Verpackung abgefüllt ist und die Verpackung eine Vorrichtung zum Verschäumen aufweist.

Es wird ein Schaum mit guter, kompakter und elastischer Schaumqualität erhalten, der auf dem Haar beim Verreiben schnell zusammenbricht und gut in das Haar einarbeitbar ist. Der Schaum ist stabil und unter leichtem Druck formbar und zerfällt erst bei intensiverem Verreiben. Die aufgeschäumte Zusammensetzung ist vorzugsweise soweit stabil, dass nach 30 min das Schaumvolumen mindestens 50%, insbesondere mindestens 75% des Schaumvolumens unmittelbar nach der Ausbringung aus der Verpackung beträgt. Das erfindungsgemäße Schaumprodukt zeichnet sich außer durch die besondere Schaumkonsistenz, - haptik und -stabilität bei einer Anwendung auf menschlichen Haaren zur Frisurerstellung insbesondere auch durch einen guten Haarglanz, gute Formerstellung, Festigung und Stabilität der Frisur und eine gute Lockendefinition und ein gutes Rückstandsverhalten aus. Aufgrund der guten Lockendefinition eignet sich die erfindungsgemäße Zusammensetzung besonders zur Behandlung von gelockten oder gewellten Haaren.

### Copolymer (A)

Das Copolymer (A) wird vorzugsweise in einer Menge von 0,1 bis 20, besonders bevorzugt von 0,5 bis 10 Gew.%, bezogen auf die verschäumbare Zusammensetzung eingesetzt. Es ist aufgebaut aus Itaconsäuremonoestern der allgemeinen Formel CH₂=C(COOR¹)CH₂COOR² wobei einer der Substituenten R¹ und R² für Wasserstoff und der andere für die Gruppe -(CH₂CH₂O)ₓ-R³ steht. X ist eine Zahl zwischen 1 und 100 vorzugsweise zwischen 10 und 40, besonders bevorzugt 20. R³ ist eine Alkylgruppe mit 8 bis 30, vorzugsweise 12 bis 20 C-Atomen, besonders bevorzugt Cetyl oder Stearyl.

Die Acrylat-Monomeren des Copolymers (A) sind vorzugsweise ausgewählt aus Acrylsäure, Methacrylsäure und deren einfachen Estern, insbesondere den Acrylsäurealkylestern und Methacrylsäurealkylestern mit 1 bis 10, vorzugsweise 1 bis 4 C-Atomen in der Alkylgruppe. Geeignete Copolymere sind z.B. Acryl- oder Methacrylsäure/Itaconsäurepolyethoxyalkylester Copolymere (INCI-Bezeichnungen: Acrylates/ Steareth-20 Itaconate Copolymer und Acrylates/Ceteth-20 Itaconate Copolymer), wie sie von der Firma National Starch/ USA unter den Bezeichnungen Structure® 2001 und Structure® 3001 vertrieben werden.

### Neutralisationsmittel

Die Säuregruppen des Copolymers (A) sind vorzugsweise durch organische oder anorganische Basen zu 50 bis 100%, insbesondere zu 70 bis 100%, bezogen auf die Anzahl der freien Säuregruppen, neutralisiert. Als Neutralisationsmittel können prinzipiell alle kosmetisch verträglichen anorganischen oder organischen Basen verwendet werden. Neutralisationsmittel sind z.B. primäre oder sekundäre Amine, insbesondere Aminoalkanole mit vorzugsweise 1 bis 10 C-Atomen und 1 bis 3 Hydroxygruppen wie z.B. Aminomethylpropanol (AMP), Triethanolamin, Tetrahydroxypropylethylendiamin oder Monoethanolamin, aber auch Ammoniak oder basische Metallhydroxide, insbesondere von Alkali- oder Erdalkalimetallen wie z.B. NaOH, KOH u.a., wobei Aminoalkanole, insbesondere AMP bevorzugt sind.

### Lösungsmittelsystem

Die erfindungsgemäße verschäumbare Zusammensetzung wird in einem rein wässrigen oder in einem wässrig-alkoholischen Medium mit vorzugsweise mindestens 50 Gew.%, insbesondere mindestens 75% Gew. Wasser konfektioniert. Das Lösungsmittelsystem ist vorzugsweise in einer Menge von 50 bis 98, besonders bevorzugt von 75 bis 95 Gew.%, bezogen auf die verschäumbare Zusammensetzung (ohne Treibmittel) enthalten. Als Alkohole können insbesondere die für kosmetische Zwecke üblicherweise verwendeten niederen Alkohole mit 1 bis 4 C-Atomen, z.B. Ethanol und Isopropanol enthalten sein. Als zusätzliche Co-Solventien können organische Lösungsmittel oder ein Gemisch aus Lösungsmitteln mit einem Siedepunkt unter 400°C in einer Menge von 0,1 bis 15, bevorzugt von 1 bis 10 Gew.% enthalten sein. Besonders geeignet als zusätzliche Co-Solventien sind unverzweigte oder verzweigte Kohlenwasserstoffe wie Pentan, Hexan, Isopentan und cyclische Kohlenwasserstoffe wie Cyclopentan und Cyclohexan. Weitere, besonders bevorzugte wasserlösliche Lösungsmittel sind Glycerin, Ethylenglykol und Propylenglykol.

Die verschäumbare Zusammensetzung weist einen pH-Wert von vorzugsweise größer als 7, insbesondere mindestens 7,3 auf. Besonders bevorzugt ist der pH-Bereich zwischen 7,5 und 8,5.

Bei geringeren pH-Werten können die besonderen rheologischen und haptischen Eigenschaften der Zusammensetzung bzw. des Schaums beeinträchtigt sein.

### Zusätzliches Tensid

In einer bevorzugten Ausführungsform ist in der erfindungsgemäßen verschäumbaren Zusammensetzung zusätzlich mindestens ein Tensid enthalten. Das Tensid ist vorzugsweise in einer Menge von 0,01 bis 15, besonders bevorzugt von 0,05 bis 10 Gew.%, bezogen auf die verschäumbare Zusammensetzung, enthalten. Geeignete Tenside haben emulgierende, lösungsvermittelnde, schaumbildende, schaumverstärkende oder haarpflegende Eigenschaften, sind vorzugsweise kationisch oder nichtionisch und haben einen HLB-Wert von maximal 20, vorzugsweise von 5 bis 18. In einer besonders bevorzugten Ausführungsform ist sowohl ein nichtionisches als auch ein kationisches Tensid enthalten.

Bevorzugte nichtionische Tenside sind ethoxylierte Tenside, wobei die Anzahl der Ethylenoxid-Einheiten zwischen 1 bis 1000, bevorzugt zwischen 1 bis 300, besonders bevorzugt zwischen 1 und 15 liegt. Bevorzugt werden Fettsäureglyceridethoxylate, Fettalkoholethoxylate, Fettaminethoxylate, Fettsäurealkanolamidethoxylate und Fettsäureesterethoxylate mit jeweils 1 bis 50 Ethylenoxideinheiten. Beispiele für geeignete Fettalkoholethoxylate sind oxethylierter Lauryl-, Tetradecyl-, Cetyl-, Oleyl- oder Stearylalkohol, die allein oder im Gemisch eingesetzt werden können, sowie Fettalkohole von oxethyliertem Lanolin oder oxethyliertes Lanolin. Auch die ethoxylierten Fettalkohole, die unter der Typenbezeichnung Dehydol® von der Firma Henkel oder unter der Typenbezeichnung Brij® von der Firma ICI Surfactants vertrieben werden, sind für das erfindungsgemäße Haarbehandlungsmittel geeignet. Unter den Fettsäureesterethoxylaten sind vor allem Diglyceridethoxylate zu nennen, z.B. mit 25 Ethylenoxid-Einheiten ethoxyliertes Rizinusöl mit dem INCI-Namen PEG-25 Hydrogenated Castor Oil (Arlatone® G), mit 35 Ethylenoxid-Einheiten ethoxyliertes Rizinusöl mit dem INCI-Namen PEG-35 Castor Oil (Cremophor® El) oder mit 40 Ethylenoxid-Einheiten ethoxyliertes hydriertes Rizinusöl mit dem INCI-Namen PEG-40 Hydrogenated Castor Oil (Cremophor® RH 410). Weitere geeignete nichtionische Tenside sind ethoxylierte Fettsäurezuckerester, insbesondere ethoxylierte Sorbitanfettsäureester, die als Polysorbate bekannt sind, aber auch nicht ethoxylierte Tenside, wie die Fettsäurezuckerester, die von der Firma ICI Surfactants unter dem Handelsnamen Tween® und Arlacel® vertrieben werden sowie die Alkylpolyglycoside, die von der Firma Cognis unter dem Handelsnamen Plantaren® oder Plantacare® oder von der Firma Seppic unter dem Handelsnamen Oramix® vertrieben werden.

Geeignete kationische Tenside weisen eine quaternäre Ammoniumgruppe auf und können durch die allgemeine Formel (I) dargestellt werden,

N⁽⁺⁾R¹R²R³R⁴ X⁽⁻⁾ (I)

wobei R1 bis R4 unabhängig voneinander Alkylgruppen, Arylgruppen, Alkoxygruppen, Polyoxyalkylengruppen, Alkylamidogruppen, Hydroxyalkylgruppen, Arylgruppen oder Alkarylgruppen mit 1 bis 22 C-Atomen bedeuten und X⁽⁻⁾ ein Anion darstellt, z.B. ein Halogen, Acetat, Phosphat, Nitrat oder Alkylsulfat, vorzugsweise ein Chlorid oder Bromid. Die aliphatischen Gruppen können zusätzlich zu den C-Atomen und den Wasserstoffatomen auch Querverbindungen oder andere Gruppen wie beispielsweise weitere Aminogruppen enthalten. Beispiele für geeignete kationische Tenside sind die Chloride oder Bromide von Alkyldimethylbenzylammoniumsalzen, Alkyltrimethylammoniumsalze, z.B. Cetyltrimethylammoniumchlorid oder -bromid, Tetradecyltrimethylammoniumchlorid oder -bromid, Alkyldimethylhydroxyethylammoniumchloride oder -bromide, die Dialkyldimethylammoniumchloride oder -bromide, Alkylpyridiniumsalze, z.B. Lauryl- oder Cetylpyridiniumchlorid, Alkylamidoethyltrimethylammoniumethersulfate sowie Verbindungen mit kationischem Charakter wie Aminoxide, z.B. Alkylmethylaminoxide oder Alkylaminoethyldimethylaminoxide. Besonders bevorzugt ist Cetyltrimethylammoniumchlorid.

Die verschäumbare Zusammensetzung weist vorzugsweise mindestens eine, mehrere oder alle der folgenden rheologischen Eigenschaften auf:
- Viskosität von mindestens 50 mPa s, insbesondere mindestens 100 mPa s, vorzugsweise mindestens 250 mPa s (gemessen bei 25°C und einer Schergeschwindigkeit von 50 s⁻¹);
- Auftreten eines Maximums in der Viskositätskurve bei ansteigender Schergeschwindigkeit; das Maximum liegt typischerweise bei Schergeschwindigkeiten im Bereich von 0,1 bis 5,0, vorzugsweise von 0,15 bis 2,0 s⁻¹ bei einer Temperatur von 25°C;
- Starke Viskositätsabnahme bei steigender Schergeschwindigkeit (shear thinning); die Viskositätsabnahme kann mehr als 25%, insbesondere mehr als 50% bei von 20 s⁻¹ auf 100 s⁻¹ steigendem Schergefälle betragen;
- ein Elastizitätsmodul G' von mindestens 60 Pa, insbesondere mindestens 80 Pa, vorzugsweise mindestens 100 Pa bei 25°C einer Messfrequenz von 1 Hz und einer Scherspannung von 2 Pa.

Die Viskositätsmessungen können mit einem Bohlin Rheometer CS, Messsystem DG40/50, C25 bei 25°C erfolgen. Die Messung des Elastizitätsmoduls kann durch Oszillationsmessungen mit einem Bohlin Rheometer CS, Messsystem FL/DG (Flügeldrehkörper) bei 25°C und einer Scherspannung von 2 Pa erfolgen. Das Maximum in der Viskositätskurve liegt typischerweise bei Schergeschwindigkeiten im Bereich von 0,1 bis 5,0, vorzugsweise von 0,15 bis 2,0 s⁻¹ bei einer Temperatur von 25°C. Die Fließkurve weist bei der Scherung, bei der die Viskosität ein Maximum erreicht, einen Wendepunkt auf. Eine weitere Besonderheit ist, dass sich die Viskositätskurve für ansteigende Schergeschwindigkeiten mit derjenigen für fallende Schergeschwindigkeiten kreuzt. Der Kreuzungspunkt liegt typischerweise bei Schergeschwindigkeiten im Bereich von 0,05 bis 3, vorzugsweise 0,1 bis 2 s⁻¹ . Bei hohen Schergeschwindigkeiten liegt die Hinkurve oberhalb der Rückkurve und die Zusammensetzung ist thixotrop. Bei niedrigen Schergeschwindigkeiten liegt die Hinkurve unterhalb der Rückkurve und die Zusammensetzung ist anti-thixotrop (rheopex). Gemessen werden können die Viskositäts- und Fließkurven mit einem Rheometer Bohlin CS System, wobei unter isothermen Bedingungen (25°C) die Schergeschwindigkeit mit linearer Geschwindigkeit von 0 bis auf einen Maximalwert z.B. ca. 100 s⁻¹ gesteigert und anschließend in gleicher Weise wieder auf den Nullpunkt gesenkt wird.

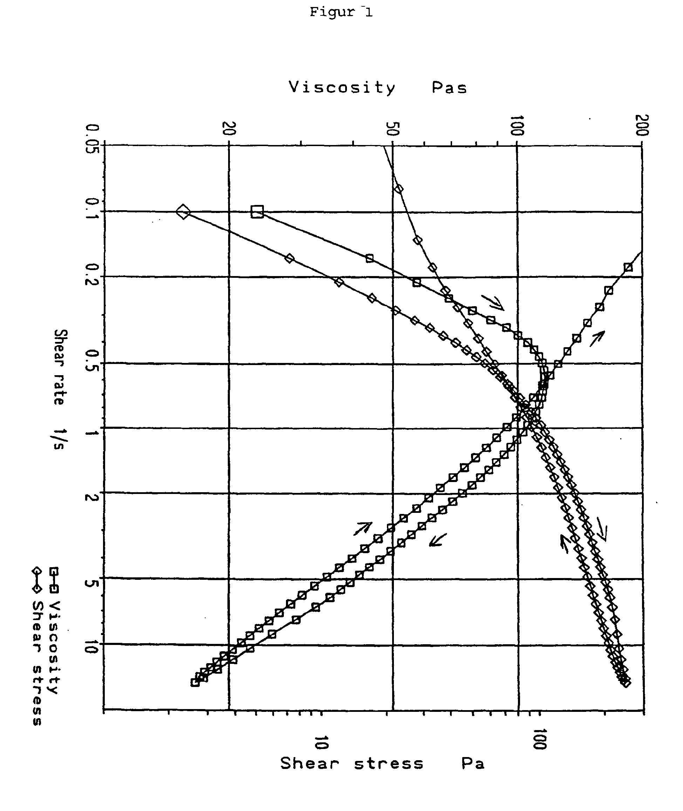

Figur 1 zeigt ein typisches Beispiel eines Messdiagramms, aufgenommen mit einem Bohlin CS System Rheometer bei einer Temperatur von 25°C für die Zusammensetzung gemäß Beispiel 10. Aufgetragen sind die Viskosität (viscosity, linke Achse, Messwerte dargestellt als Quadrate) und die Scherspannung (shear stress, rechte Achse, Messwerte dargestellt als Rauten) gegen die Schergeschwindigkeit (shear rate). Die Pfeile an den Kurven geben die Richtung der Änderung der Schergeschwindigkeit an. Bei einer Schergeschwindigkeit von ca. 0,6 s⁻¹ hat die Viskositätskurve ein Maximum und die Fließkurve (shear stress) einen Wendepunkt. Bei einer Schergeschwindigkeit von ca. 0,7 s⁻¹ schneiden sich die Hin- und die Rückkurven.

### Zusätzlicher haarfestigender Wirkstoff

In einer bevorzugten Ausführungsform ist in der verschäumbaren Zusammensetzung zusätzlich zu Copolymer (A) mindestens ein haarfestigender Wirkstoff in einer Menge von vorzugsweise 0,01 bis 15, besonders bevorzugt von 0,5 bis 10 Gew.% enthalten. Bei dem haarfestigenden Wirkstoff kann es sich um ein Mono- Di- oder Oligosaccharid oder deren Gemisch oder um ein haarfestigendes Polymer handeln. Saccharide sind z.B. Glucose, Fructose, Galactose, Maltose usw.. Haarfestigende Polymere können nichtionisch, anionisch, kationisch, zwitterionisch oder amphoter sein, sofern sie mit den übrigen Bestandteilen der Zusammensetzung verträglich sind. Vorzugsweise werden nichtionische, anionische oder amphotere Polymere eingesetzt. Es kann ein synthetisches oder ein natürliches Polymer sein. Unter natürlichen Polymeren werden auch chemisch modifizierte Polymere natürlichen Ursprungs verstanden. Bevorzugt sind insbesondere solche Polymere, die eine ausreichende Löslichkeit in Wasser, Alkohol oder Wasser/Alkohol-Gemischen besitzen, um in dem erfindungsgemäßen Mittel in vollständig gelöster Form vorzuliegen. Unter haarfestigenden Polymeren werden erfindungsgemäß solche Polymere verstanden, die bei Anwendung in 0,01 bis 5%iger wässriger, alkoholischer oder wässrig-alkoholischer Lösung oder Dispersion in der Lage sind, auf dem Haar eine spür- oder meßbare haarfestigende Wirkung zu erzielen.

Geeignete synthetische, nichtionische haarfestigende Polymere sind Homo- oder Copolymere, die aus mindestens einem der folgenden Monomere aufgebaut sind: Vinylpyrrolidon, Vinylcaprolactam, Vinylester wie z.B. Vinylacetat, Vinylalkohol, Acrylamid, Methacrylamid, Alkyl- und Dialkylacrylamid, Alkylund Dialkylmethacrylamid, Dialkylaminoalkylmethacrylamid, Dialkylaminoalkylacrylamid, Alkylacrylat, Alkylmethacrylat, Propylenglykol oder Ethylenglykol, wobei die Alkylgruppen dieser Monomere vorzugsweise C1- bis C7-Alkylgruppen, besonders bevorzugt C1- bis C3-Alkylgruppen sind. Geeignet sind z.B. Homopolymere des Vinylcaprolactams, des Vinylpyrrolidons oder des N-Vinylformamids. Weitere geeignete haarfestigende Polymere sind z.B. Copolymerisate aus Vinylpyrrolidon und Vinylacetat, Terpolymere aus Vinylpyrrolidon, Vinylacetat und Vinylpropionat, Terpolymere aus Vinylpyrrolidon, Vinylcaprolactam und Dialkylaminoalkyl(meth)acrylat, Terpolymere aus Vinylpyrrolidon, Vinylcaprolactam und Dialkylaminoalkyl(meth)-acrylamid, Polyacrylamide, Polyvinylalkohole, sowie haarfestigende Polyethylenglykol/Polypropylenglykol Copolymere. Besonders bevorzugte nichtionische Polymere sind Polyvinylpyrrolidon und Polyvinylpyrrolidon/Vinylacetat Copolymere.

Geeignete natürliche filmbildende Polymere sind z.B. hydroxyalkylierte Polysaccharide, insbesondere Hydroxyalkylcellulose oder Hydroxyalkylguar mit vorzugsweise 2 oder 3 C-Atomen in der Alkylgruppe.

Geeignete anionische haarfestigende Polymere können natürliche oder synthetische Homo- oder Copolymere mit Säuregruppen enthaltenden Monomereinheiten sein, welche gegebenenfalls mit Comonomeren, die keine Säuregruppen enthalten, copolymerisiert sind. Die Säuregruppen sind vorzugsweise ausgewählt aus -COOH, -SO₃H, -OSO₃H, -OPO₂H und -OPO₃H₂, von denen die Carbonsäuregruppen bevorzugt sind. Die Säuregruppen können unneutralisiert, teilweise oder vollständig neutralisiert vorliegen. Sie liegen vorzugsweise zu 50 bis 100% in anionischer bzw. neutralisierter Form vor. Als Neutralisationsmittel können die oben genannten Neutralisationsmittel verwendet werden. Geeignete Monomere sind ungesättigte, radikalisch polymerisierbare Verbindungen, welche mindestens eine Säuregruppe tragen, insbesondere Carboxyvinylmonomere. Geeignete Säuregruppen enthaltende Monomere sind beispielsweise Acrylsäure, Methacrylsäure, Crotonsäure, Maleinsäure bzw. Maleinsäureanhydrid oder deren Monoester, Aldehydocarbonsäuren oder Ketocarbonsäuren.

Nicht mit Säuregruppen substituierte Comonomere sind beispielsweise Acrylamid, Methacrylamid, Alkyl- und Dialkylacrylamid, Alkyl- und Dialkylmethacrylamid, Alkylacrylat, Alkylmethacrylat, Vinylcaprolacton, Vinylpyrrolidon, Vinylester, Vinylalkohol, Propylenglykol oder Ethylenglykol, aminsubstituierte Vinylmonomere wie z.B. Dialkylaminoalkylacrylat, Dialkylaminoalkylmethacrylat, Monoalkylaminoalkylacrylat und Monoalkylaminoalkylmethacrylat, wobei die Alkylgruppen dieser Monomere vorzugsweise C1- bis C7-Alkylgruppen, besonders bevorzugt C1- bis C3-Alkylgruppen sind.

Geeignete anionische Polymere sind insbesondere Copolymere der Acrylsäure oder Methacrylsäure mit Monomeren ausgewählt aus Acrylsäure- oder Methacrylsäureestern, Acrylamiden, Methacrylamiden und Vinylpyrrolidon, Homopolymere der Crotonsäure sowie Copolymere der Crotonsäure mit Monomeren ausgewählt aus Vinylestern, Acrylsäure- oder Methacrylsäureestern, Acrylamiden und Methacrylamiden. Ein geeignetes natürliches Polymer ist beispielsweise Schellack.

Bevorzugte anionische Polymere sind vernetzte oder unvernetzte Vinylacetat/Crotonsäure Copolymere. Ebenso bevorzugt sind partialveresterte Copolymere zwischen Vinylmethylether und Maleinsäureanhydrid. Weitere geeignete anionische Polymere sind z.B. Terpolymere aus Acrylsäure, Alkylacrylat und N-Alkylacrylamid, insbesondere Acrylsäure/Ethylacrylat/N-t-Butylacrylamid Terpolymere oder Terpolymere aus Vinylacetat, Crotonat und Vinylalkanoat, insbesondere Vinylacetat/Crotonat/Vinylneodecanoat Copolymere.

Amphotere oder zwitterionische Polymere können Homo- oder Copolymere sein, welche sowohl kationische oder kationisierbare Amin- bzw. Ammoniumgruppen als auch anionische oder anionisierbare Säuregruppen enthalten, wobei die kationischen bzw. kationisierbaren Gruppen entweder in der Polymerkette oder vorzugsweise als Substituent an einem oder mehreren der Monomeren enthalten sind. Das amphotere Polymer kann mit neutralen Comonomeren copolymerisiert sein, die weder kationische bzw. kationisierbare Gruppen noch anionische bzw. anionisierbare Gruppen enthalten. Derartige neutrale Comonomere sind z.B. Acrylamid, Methacrylamid, Alkyl- und Dialkylacrylamid, Alkyl- und Dialkylmethacrylamid, Alkylacrylat, Alkylmethacrylat, Vinylcaprolacton, Vinylpyrrolidon, Vinylcaprolactam, Vinylester, Vinylalkohol, Propylenglykol oder Ethylenglykol, wobei die Alkylgruppen dieser Monomere vorzugsweise C1- bis C7-Alkylgruppen, besonders bevorzugt C1- bis C3-Alkylgruppen sind.

Das amphotere Polymer ist vorzugsweise ein Copolymer, welches gebildet ist aus mindestens einer ersten Monomerart, welche mindestens eine neutralisierte oder nicht neutralisierte Säuregruppe aufweist und mindestens einer zweiten Monomerart, welche mindestens eine neutralisierte oder nicht neutralisierte basische Gruppe aufweist. Geeignete Monomere des amphoteren Polymers, welche Säuregruppen aufweisen, sind ungesättigte, radikalisch polymerisierbare Verbindungen, welche mindestens eine Säuregruppe, z.B. eine Carbonsäuregruppe tragen, insbesondere Carboxyvinylmonomere wie z.B. Acrylsäure, Methacrylsäure, Crotonsäure oder Maleinsäure bzw. deren Monoester, von denen Acrylsäure und Methacrylsäure bevorzugt sind. Geeignete Monomere des amphoteren Polymers, welche neutralisierte oder nicht neutralisierte basische Gruppen aufweisen, sind ungesättigte, radikalisch polymerisierbare Verbindungen. Als basische Gruppen kommen insbesondere primäre, sekundäre oder tertiäre Amine in Betracht, wobei das N-Atom auch Teil eines Ringes sein kann. Beispiele für derartige amphotere Copolymere sind Copolymere gebildet aus Alkylacrylamid, Alkylaminoalkylmethacrylat und ein, zwei oder mehr Monomeren bestehend aus Acrylsäure, Methacrylsäure und deren einfachen Alkylestern, wobei mindestens eines der Monomere eine Säuregruppe trägt. Ein bevorzugtes amphoteres Polymer ist ein Copolymer aus Octylacrylamid, N-tert.-Butylaminoethylmethacrylat und zwei oder mehr Monomeren ausgewählt aus Acrylsäure, Methacrylsäure und deren Alkylestern, wobei mindestens eines der Monomere eine Säuregruppe trägt. Entsprechende Handelsprodukte sind Amphomer® oder Amphomer® LV-71.

Weitere Beispiele für haarfestigende Polymere sind Copolymere von Acrylsäure, Methylacrylat und Methacrylamidopropyltrimethylammoniumchlorid (INCI-Bezeichnung: Polyquaternium-47), Copolymere aus Acrylamidopropyltrimethylammoniumchlorid und Acrylaten, oder Copolymere aus Acrylamid, Acrylamidopropyltrimethylammoniumchlorid, 2-Amidopropylacrylamidsulfonat und Dimethylaminopropylamin (INCI-Bezeichnung: Polyquaternium-43). Polymere mit Betaingruppen tragenden Monomeren sind z.B. Copolymere aus Methacryloylethylbetain und zwei oder mehr Monomeren von Acrylsäure oder deren einfachen Estern, bekannt unter der INCI-Bezeichnung Methacryloyl Ethyl Betaine/Acrylates Copolymer.

### Zusätzliche haarfärbende Stoffe

Bei einer besonderen Ausführungsform handelt es sich um ein haarfarbveränderndes Produkt, insbesondere einen Farbschaumfestiger. Die Zusammensetzung enthält dann mindestens einen haarfärbenden Stoff. Hierbei kann es sich um lösliche, organische Farbstoffe, insbesondere um sogenannte direktziehende Farbstoffe oder auch um unlösliche anorganische oder organische Pigmente handeln. Die Gesamtmenge an Farbstoffen beträgt in dem erfindungsgemäßen Mittel etwa 0,01 bis 15 Gew.%, vorzugsweise etwa 0,2 bis 7 Gew.%, bezogen auf die verschäumbare Zusammensetzung. Mit direktziehenden Farbstoffen können semipermanente Färbungen, mit Pigmenten können temporäre Färbungen erhalten werden. Unter semi-permanenter Haarfärbung wird eine Farbänderung von menschlichen Haaren verstanden, die mehrere (mindestens zwei) Haarwäschen übersteht. Unter temporärer Haarfärbung wird eine Farbänderung von menschlichen Haaren verstanden, die bis zur nächsten Haarwäsche hält und durch Waschen der Haare mit üblichen Shampoos wieder entfernt werden kann. Geeignete direktziehende Farbstoffe sind z.B. Triphenylmethanfarbstoffe, aromatische Nitrofarbstoffe, Azofarbstoffe, Chinonfarbstoffe, kationische oder anionische Farbstoffe. Geeignet sind:
Nitrofarbstoffe (blau):
   1,4-Bis[(2-hydroxyethyl)amino]-2-nitrobenzol, 1-(2-Hydroxyethyl)amino-2-nitro-4-[di(2-hydroxyethyl)amino]-benzol (HC Blue No. 2), 1-Amino-3-methyl-4-[(2-hydroxyethyl)amino]-6-nitrobenzol (HC Violet No. 1), 4-[Ethyl-(2-hydroxyethyl)-amino]-1-[(2-hydroxyethyl)amino]-2-nitrobenzol-hydrochlorid (HC Blue No. 12), 4-[Di(2-hydroxyethyl)amino]-1-[(2-methoxyethyl)amino]-2-nitrobenzol (HC Blue No. 11), 1-[(2,3-Dihydroxypropyl)amino]-4-[methyl-(2-hydroxyethyl)amino]-2-nitrobenzol (HC Blue No. 10), 1-[(2,3-Dihydroxypropyl)-amino]-4-[ethyl-(2-hydroxyethyl)amino]-2-nitrobenzol-hydrochlorid (HC Blue No. 9), 1-(3-Hydroxypropylamino)-4-[di(2-hydroxyethyl)amino]-2-nitrobenzol (HC Violet No. 2), 1-Methylamino-4-[methyl-(2,3-dihydroxypropyl)amino]-2-nitrobenzol (HC Blue No. 6), 2-((4-Amino-2-nitrophenyl)amino)-5-dimethylamino-benzoesäure (HC Blue No. 13), 1-(2-Aminoethylamino)-4-[di(2-hydroxyethyl)amino]-2-nitrobenzol, 4-(Di(2-hydroxyethyl)amino)-2-nitro-1-phenylamino-benzol.
Nitrofarbstoffe (rot):
   1-Amino-4-[(2-hydroxyethyl)amino]-2-nitrobenzol (HC Red No. 7), 2-Amino-4,6-dinitro-phenol, 1,4-Diamino-2-nitrobenzol (CI76070), 4-Amino-2-nitro-diphenylamin (HC Red No. 1), 1-Amino-4-[di(2-hydroxyethyl)amino]-2-nitrobenzol-hydrochlorid (HC Red No. 13), 1-Amino-5-chlor-4-[(2-hydroxyethyl)amino]-2-nitrobenzol, 4-Amino-1-[(2-hydroxyethyl)amino]-2-nitrobenzol (HC Red No. 3), 4-((2-Hydroxyethyl)methylamino)-1-(methylamino)-2-nitrobenzol, 1-Amino-4-((2,3-dihydroxypropyl)amino)-5-methyl-2-nitrobenzol, 1-Amino-4-(methylamino)-2-nitrobenzol, 4-Amino-2-nitro-1-((prop-2-en-1-yl)-amino)-benzol, 4-Amino-3-nitrophenol, 4-[(2-Hydroxyethyl)-amino]-3-nitrophenol, 4-[(2-Nitrophenyl)amino]phenol (HC Orange No. 1), 1-[(2-Aminoethyl)amino]-4-(2-hydroxyethoxy)-2-nitrobenzol (HC Orange No. 2), 4-(2,3-Dihydroxypropoxy)-1-[(2-hydroxyethyl)amino]-2-nitrobenzol (HC Orange No. 3), 1-Amino-5-chlor-4-[(2,3-dihydroxypropyl)amino]-2-nitrobenzol (HC Red No. 10), 5-Chlor-1,4-[di(2,3-dihydroxypropyl)amino] - 2-nitrobenzol (HC Red No. 11), 2-[(2-Hydroxyethyl)amino]-4,6-dinitro-phenol, 4-Ethylamino-3-nitrobenzoesäure, 2-[(4-Amino-2-nitrophenyl)amino]-benzoesäure, 2-Chlor-6-ethylamino-4-nitrophenol, 2-Amino-6-chlor-4-nitrophenol, 4-[(3-Hydroxypropyl)amino]-3-nitrophenol, 2,5-Diamino-6-nitropyridin, 6-Amino-3-((2-hydroxyethyl)amino)-2-nitropyridin, 3-Amino-6-((2-hydroxyethyl)amino)-2-nitropyridin, 3-Amino-6-(ethylamino)-2-nitropyridin, 3-((2-Hydroxyethyl)amino)-6-(methylamino)-2-nitropyridin, 3-Amino-6-(methylamino)-2-nitropyridin, 6-(Ethylamino)-3-((2-hydroxyethyl)amino)-2-nitropyridin, 1,2,3,4-Tetrahydro-6-nitrochinoxalin, 7-Amino-3,4-dihydro-6-nitro-2H-1,4-benzoxazin (HC Red No. 14).
Nitrofarbstoffe (gelb):
   1,2-Diamino-4-nitrobenzol (CI76020), 1-Amino-2-[(2-hydroxyethyl)amino]-5-nitrobenzol (HC Yellow No. 5), 1-(2-Hydroxyethoxy)-2-[(2-hydroxyethyl)amino]-5-nitrobenzol (HC Yellow No. 4), 1-[(2-Hydroxyethyl)amino]-2-nitrobenzol (HC Yellow No. 2), 2-(Di(2-hydroxyethyl)amino)-5-nitrophenol, 2-[(2-Hydroxyethyl)amino]-1-methoxy-5-nitrobenzol, 2-Amino-3-nitrophenol, 1-Amino-2-methyl-6-nitrobenzol, 1-(2-Hydroxyethoxy)-3-methylamino-4-nitrobenzol, 2,3-(Dihydroxypropoxy)-3-methylamino-4-nitrobenzol, 2-[(2-Hydroxyethyl)amino]-5-nitrophenol (HC Yellow No. 11), 3-[(2-Aminoethyl)amino]-1-methoxy-4-nitrobenzol-hydrochlorid (HC Yellow No.9), 1-[(2-Ureidoethyl)amino]-4-nitrobenzol, 4-[(2,3-Dihydroxypropyl)-amino]-3-nitro-1-trifluormethyl-benzol (HC Yellow No. 6), 1-Chlor-2,4-bis[(2-hydroxyethyl)amino]-5-nitrobenzol (HC Yellow No. 10), 1-Amino-4-((2-aminoethyl)amino)-5-methyl-2-nitrobenzol, 4-[(2-Hydroxyethyl)amino]-3-nitro-1-methylbenzol, 1-Chlor-4-[(2-hydroxyethyl)amino]-3-nitrobenzol (HC Yellow No. 12), 4-[(2-Hydroxyethyl)amino]-3-nitro-1-trifluormethyl-benzol (HC Yellow No. 13), 4-[(2-Hydroxyethyl)-amino]-3-nitro-benzonitril (HC Yellow No. 14), 4-[(2-Hydroxyethyl)amino]-3-nitro-benzamid (HC Yellow No. 15) 3-((2-Hydroxyethyl)amino)-4-methyl-1-nitrobenzol, 4-Chlor-3-((2-hydroxyethyl)amino)-1-nitrobenzol.
Chinonfarbstoffe:
   1,4-Di[(2,3-dihydroxypropyl)amino]-9,10-anthrachinon, 1,4-Di[(2-hydroxyethyl)amino]-9,10-anthrachinon (CI61545, Disperse Blue 23), 1-[(2-Hydroxyethyl)amino]-4-methylamino-9,10-anthrachinon (CI61505, Disperse Blue No. 3), 2-[(2-Aminoethyl)amino]-9,10-anthrachinon (HC Orange No. 5), 1-Amino-4-hydroxy-9,10-anthrachinon (CI60710, Disperse Red 15), l-Hydroxy-4-[(4-methyl-2-sulfophenyl)amino]-9,10-anthrachinon, 7-Beta-D-glucopyranosyl-9,10-dihydro-1-methyl-9,10-dioxo-3,5,6,8-tetrahydroxy-2-anthracencarbonsäure (CI75470, Natural Red 4), 1-[(3-Aminopropyl)amino]-4-methylamino-9,10-anthrachinon (HC Blue No. 8), 1-[(3-Aminopropyl)-amino]-9,10-anthrachinon (HC Red No. 8), 1,4-Diamino-2-methoxy-9,10-anthrachinon (CI62015, Disperse Red No. 11, Solvent Violet No. 26), 1,4-Dihydroxy-5,8-bis[(2-hydroxyethyl)amino]-9,10-anthrachinon (CI62500, Disperse Blue No. 7, Solvent Blue No. 69), 1,4-Diamino-9,10-anthrachinon (CI61100, Disperse Violet No. 1), 1-Amino-4-(methylamino)-9,10-anthrachinon (CI61105, Disperse Violet No. 4, Solvent Violet No. 12), 2-Hydroxy-3-methoxy-1,4-naphthochinon, 2,5-Dihydroxy-1,4-naphthochinon, 2-Hydroxy-3-methyl-1,4-naphthochinon, N-(6-((3-Chlor-4-(methylamino)phenyl)imino)-4-methyl-3-oxo-1,4-cyclohexadien-1-yl)harnstoff (HC Red No. 9), 2-((4-(Di(2-hydroxyethyl)amino)phenyl)amino)-5-((2-hydroxyethyl)amino)-2,5-cyclohexadien-1,4-dion (HC Green No. 1), 5-Hydroxy-1,4-naphthochinon (CI75500, Natural Brown No. 7), 2-Hydroxy-1,4-naphthochinon (CI75480, Natural Orange No. 6), 1,2-Dihydro-2-(1,3-dihydro-3-oxo-2H-indol-2-yliden)-3Hindol-3-on (CI73000), 4-((5-((2-Hydroxyethyl)amino-1-methyl-1H-pyrazol-4-yl)imino)-4,5-dihydro-5-((2-hydroxyethyl)-imino)-1-methyl-1H-Pyrazol-sulfat(1:1),hydrat(1:1).
Basische Farbstoffe:
   9-(Dimethylamino)-benzo[a]phenoxazin-7-ium-chlorid (CI51175; Basic Blue No. 6), Di[4-(diethylamino)phenyl][4-(ethylamino)naphthyl]carbenium-chlorid (CI42595; Basic Blue No. 7), Di-(4-(dimethylamino)phenyl)-(4-(methyl-phenylamino)-naphthalin-1-yl)carbenium-chlorid (CI42563; Basic Blue No. 8), 3,7-Di(dimethylamino)phenothiazin-5-ium-chlorid (CI52015 Basic Blue No. 9), Di[4-(dimethylamino)phenyl][4-(phenylamino)naphthyl]carbenium-chlorid (CI44045; Basic Blue No. 26), 2-[(4-(Ethyl(2-hydroxyethyl)amino)phenyl)azo]-6-methoxy-3-methyl-benzothiazolium-methylsulfat (CI11154; Basic Blue No. 41), 8-Amino-2-brom-5-hydroxy-4-imino-6-[(3-(trimethylammonio)phenyl)amino]-1(4H)-naphthalinon-chlorid (CI56059; Basic Blue No. 99), Bis[4-(dimethylamino)phenyl]-[4-(methylamino)phenyl]carbenium-chlorid (CI42535; Basic Violet No. 1), Tri(4-amino-3-methylphenyl)carbenium-chlorid (CI42520; Basic Violet No. 2), Tris[4-(dimethylamino)-phenyl]carbenium-chlorid (CI42555; Basic Violet No. 3), 2-[3,6-(Diethylamino)dibenzopyranium-9-yl]-benzoesäurechlorid (CI45170; Basic Violet No. 10), Di(4-aminophenyl)(4-amino-3-methylphenyl)carbeniumchlorid (CI42510 Basic Violet No. 14), 1,3-Bis[(2,4-diamino-5-methylphenyl)azo]-3-methylbenzol (CI21010; Basic Brown No. 4), 1-[(4-Aminophenyl)azo]-7-(trimethylammonio)-2-naphthol-chlorid (CI12250; Basic Brown No. 16), 3-[(4-Amino-2,5-dimethoxyphenyl)azo]-N,N,N-trimethylbenzolaminiumchlorid (CI112605, Basic Orange No. 69), 1-[(4-Amino-2-nitrophenyl)azo]-7-(trimethylammonio)-2-naphtholchlorid, 1-[(4-Amino-3-nitrophenyl)azo]-7-(trimethylammonio)-2-naphthol-chlorid (CI12251; Basic Brown No. 17), 3,7-Diamino-2,8-dimethyl-5-phenylphenazinium-chlorid (CI50240; Basic Red No. 2), 1,4-Dimethyl-5-[(4-(dimethylamino)phenyl)azo]-1,2,4-triazolium-chlorid (CI11055; Basic Red No. 22), 2-Hydroxy-1-[(2-methoxyphenyl)azo]-7-(trimethylammonio)-naphthalin-chlorid (CI12245; Basic Red No. 76), 2-[2-((2,4-Dimethoxyphenyl)amino)ethenyl]-1,3,3-trimethyl-3H-indol-1-ium-chlorid (CI48055; Basic Yellow No. 11), 3-Methyl-1-phenyl-4-[(3-(trimethylammonio)phenyl)azo]-pyrazol-5-on-chlorid (CI12719; Basic Yellow No. 57), Di[4-(dimethylamino)phenyl]iminomethan-hydrochlorid (CI41000; Basic Yellow No. 2), Bis[4-(diethylamino)phenyl]phenylcarbenium-hydrogensulfat (1:1) (CI42040; Basic Green No. 1), Di(4-(dimethylamino)phenyl)-phenylmethanol (CI42000; Basic Green No. 4), 1-(2-Morpholiniumpropylamino)-4-hydroxy-9,10-anthrachinon-methylsulfat, 1-[(3-(Dimethyl-propylaminium)-propyl)amino]-4-(methylamino)-9,10-anthrachinon-chlorid.
Neutrale Azofarbstoffe:
   1-(Di(2-hydroxyethyl)amino]-3-methyl-4-[(4-nitrophenyl)azo]-benzol (CI11210, Disperse Red No. 17), 1-[Di(2-hydroxyethyl)amino]-4-[(4-nitrophenyl)azo]-benzol (Disperse Black No. 9), 4-[(4-Aminophenyl)azo]-1-[di(2-hydroxyethyl)amino]-3-methylbenzol (HC Yellow No. 7), 2,6-Diamino-3-[(pyridin-3-yl)azo]-pyridin, 2-((4-(Acetylamino)phenyl)azo)-4-methylphenol (CI11855; Disperse Yellow No. 3).
Saure Farbstoffe:
   6-Hydroxy-5-[(4-sulfophenyl)azo]-2-naphthalinsulfonsäuredinatriumsalz (CI15985; Food Yellow No. 3; FD&C Yellow No. 6), 2,4-Dinitro-1-naphthol-7-sulfonsäure-dinatriumsalz (CI10316; Acid Yellow No. 1; Food Yellow No. 1), 2-(Indan-1,3-dion-2-yl)chinolin-x,x-sulfonsäure (Gemisch aus Monound Disulfonsäure) (CI47005;D&C Yellow No. 10; Food Yellow No. 13; Acid Yellow No. 3), 5-Hydroxy-1-(4-sulfophenyl)-4-[(4-sulfophenyl)azo]pyrazol-3-carbonsäure-trinatriumsalz (CI19140; Food Yellow No. 4; Acid Yellow No. 23), 9-(2-Carboxyphenyl)-6-hydroxy-3H-xanthen-3-on (CI45350; Acid Yellow No. 73; D&C Yellow No. 8), 4-((4-Amino-3-sulfophenyl)azo)benzolsulfonsäure-dinatriumsalz (CI13015, Acid Yellow No. 9), 5-[(2,4-Dinitrophenyl)amino]-2-phenylaminobenzolsulfonsäure-natriumsalz (CI10385; Acid Orange No. 3), 4-[(2,4-Dihydroxyphenyl)azol-benzolsulfonsäure Mononatriumsalz (CI14270; Acid Orange No. 6), 4-[(2-Hydroxynaphth-1-yl)azo]-benzolsulfonsäure-natriumsalz (CI15510; Acid Orange No. 7), 4-[(2,4-Dihydroxy-3-[(2,4-dimethylphenyl)azo]-phenyl)azol-benzolsulfonsäure-natriumsalz (CI20170; Acid Orange No. 24), 4-Hydroxy-3-[(4-sulfonaphth-1-yl)azo]-1-naphthalin-sulfonsäure-dinatriumsalz (CI14720; Acid Red No. 14), 4-Hydroxy-3-[(2-methoxyphenyl)azo]-1-naphthalinsulfonsäure-mononatriumsalz (CI14710; Acid Red No. 4), 6-Hydroxy-5-[(4-sulfonaphth-1-yl)azo]-2,4-naphthalin-disulfonsäuretrinatriumsalz (CI16255; Ponceau 4R; Acid Red No. 18), 3-Hydroxy-4-[(4-sulfonaphth-1-yl)azo]-2,7-naphthalin-disulfonsäure-trinatriumsalz (CI16185; Acid Red No. 27), 8-Amino-1-hydroxy-2-(phenylazo)-3,6-naphthalin-disulfonsäure-dinatriumsalz (CI17200; Acid Red No. 33), 5-(Acetylamino)-4-hydroxy-3-[(2-methylphenyl)azo]-2,7-naphthalin-disulfonsäure-dinatriumsalz (CI18065; Acid Red No. 35), 2-(3-Hydroxy-2,4,5,7-tetraiod-dibenzopyran-6-on-9-yl)-benzoesäure-dinatriumsalz (CI45430; Acid Red No. 51), N-[6-(Diethylamino)-9-(2,4-disulfophenyl)-3H-xanthen-3-yliden]-N-ethylethanammonium-hydroxid, inneres Salz, Natriumsalz (CI45100; Acid Red No. 52), 8-[(4-(Phenylazo)phenyl)azo]-7-naphthol-1,3-disulfonsäure-dinatriumsalz (CI27290; Acid Red No. 73), 2',4',5',7'-Tetrabrom-3',6'-dihydroxyspiro[isobenzofuran-1(3H),9'-[9H]xanthen]-3-on-dinatriumsalz (CI45380 Acid Red No. 87), 2',4',5',7'-Tetrabrom-4,5,6,7-tetrachlor-3',6'-dihydroxyspiro[isobenzofuran-1(3H),9'[9H]xanthen]-3-on-dinatriumsalz (CI45410; Acid Red No. 92), 3',6'-Dihydroxy-4',5'-diiodospiro[isobenzofuran-1(3H),9'(9H)-xanthen]-3-on-dinatriumsalz (CI45425; Acid Red No. 95), 2-Hydroxy-3-((2-hydroxynaphth-1-yl)azo)-5-nitrobenzolsulfonsäure-mononatriumsalz (CI15685; Acid Red No. 184), (2-Sulfophenyl)di[4-(ethyl((4-sulfophenyl)methyl)amino)phenyl]-carbenium-dinatriumsalz Betain (CI42090; Acid Blue No. 9; FD&C Blue No. 1), 1,4-Bis[(2-sulfo-4-methylphenyl)amino]-9,10-anthrachinon-dinatriumsalz (CI 61570; Acid Green No. 25), Bis[4-(dimethylamino)phenyl]-(3,7-disulfo-2-hydroxynaphth-1-yl)carbenium-inneres Salz Mononatriumsalz (CI44090; Food Green No. 4; Acid Green No. 50), Bis[4-(diethylamino)-phenyl](2,4-disulfophenyl)carbenium-inneres salz, Natriumsalz (2:1) (CI42045; Food Blue No. 3; Acid Blue No. 1), Bis[4-(diethylamino)phenyl](5-hydroxy-2,4-disulfophenyl)-carbenium-inneres salz, Calciumsalz (2:1) (CI42051; Acid Blue No. 3), 1-Amino-4-(cyclohexylamino)-9,10-anthrachinon-2-sulfonsäure-natriumsalz (CI62045; Acid Blue No. 62), 1-Amino-4-(phenylamino)-9,10-anthrachinon-2-sulfonsäure (CI62055; Acid Blue No. 25), 2-(1,3-Dihydro-3-oxo-5-sulfo-2H-indol-2-yliden)-2,3-dihydro-3-oxo-1H-indol-5-sulfonsäuredinatriumsalz (CI73015; Acid Blue No. 74), 9-(2-Carboxyphenyl)-3-[(2-methylphenyl)amino]-6-[(2-methyl-4-sulfophenyl)amino]xanthylium-inneres Salz, mononatriumsalz (CI45190; Acid Violet No. 9), 1-Hydroxy-4-[(4-methyl-2-sulfophenyl)amino]-9,10-anthrachinon-natriumsalz (CI60730; D&C Violett No. 2; Acid Violet No. 43), Bis[3-nitro-4-[(4-phenylamino)-3-sulfo-phenylamino]-phenyl]-sulfon (CI10410; Acid Brown No. 13), 5-Amino-4-hydroxy-6-[(4-nitrophenyl)-azo]-3-(phenylazo)-2,7-naphthalin-disulfonsäure Dinatriumsalz (CI20470; Acid Black No. 1), 3-Hydroxy-4-[(2-hydroxynaphth-1-yl)azo]-7-nitro-1-naphthalin-sulfonsäure-chromkomplex (3:2) (CI15711; Acid Black No. 52), 3-[(2,4-Dimethyl-5-sulfophenyl)azo]-4-hydroxy-1-naphthalin-sulfonsäure-dinatriumsalz (CI14700; Food Red No. 1; Ponceau SX; FD&C Red No. 4), 4-(Acetylamino)-5-hydroxy-6-[(7-sulfo-4-[(4-sulfophenyl)azo]naphth-1-yl)azo]-1,7-naphthalindisulfonsäure-tetranatriumsalz (CI28440; Food Black No. 1), 3-Hydroxy-4-(3-methyl-5-oxo-1-phenyl-4,5-dihydro-1H-pyrazol-4-ylazo)-naphthalin-1-sulfonsäure-natriumsalz, Chrom-Komplex (Acid Red No. 195).

Weitere zur Haarfärbung bekannte und übliche Farbstoffe, die in dem erfindungsgemäßen Färbemittel enthalten sein können, sind unter anderem in E. Sagarin, "Cosmetics, Science and Technology", Interscience Publishers Inc., New York (1957), Seiten 503 ff. sowie H. Janistyn, "Handbuch der Kosmetika und Riechstoffe", Band 3 (1973), Seiten 388 ff. und K. Schrader "Grundlagen und Rezepturen der Kosmetika", 2. Auflage (1989), Seiten 782-815 beschrieben.

Geeignete haarfärbende Pigmente sind im Anwendungsmedium praktisch unlösliche Farbmittel und können anorganisch oder organisch sein. Auch anorganisch-organische Mischpigmente sind möglich. Bei den Pigmenten handelt es sich vorzugsweise nicht um Nanopigmente. Die bevorzugte Teilchengröße beträgt 1 bis 200 µm, insbesondere 3 bis 150 µm, besonders bevorzugt 10 bis 100 µm. Bevorzugt sind anorganische Pigmente. Die anorganischen Pigmente können natürlichen Ursprungs sein, beispielsweise hergestellt aus Kreide, Ocker, Umbra, Grünerde, gebranntem Terra di Siena oder Graphit. Bei den Pigmenten kann es sich um Weißpigmente wie z.B. Titandioxid oder Zinkoxid, um Schwarzpigmente wie z.B. Eisenoxidschwarz, Buntpigmente wie z.B. Ultramarin oder Eisenoxidrot, um Glanzpigmente, Metalleffekt-Pigmente, Perlglanzpigmente sowie um Fluoreszenz- oder Phosphoreszenzpigmente handeln, wobei vorzugsweise mindestens ein Pigment ein farbiges, nicht-weißes Pigment ist. Geeignet sind Metalloxide, - hydroxide und -oxidhydrate, Mischphasenpigmente, schwefelhaltige Silicate, Metallsulfide, komplexe Metallcyanide, Metallsulfate, -chromate und -molybdate sowie die Metalle selbst (Bronzepigmente). Geeignet sind insbesondere Titandioxid (CI 77891), schwarzes Eisenoxid (CI 77499), gelbes Eisenoxid (CI 77492), rotes und braunes Eisenoxid (CI 77491), Manganviolett (CI 77742), Ultramarine (Natrium-Aluminiumsulfosilikate, CI 77007, Pigment Blue 29), Chromoxidhydrat (CI77289), Eisenblau (Ferric Ferrocyanide, CI77510), Carmine (Cochineal). Besonders bevorzugt sind Pigmente auf Mica- bzw. Glimmerbasis welche mit einem Metalloxid oder einem Metalloxychlorid wie Titandioxid oder Wismutoxychlorid sowie gegebenenfalls weiteren farbgebenden Stoffen wie Eisenoxiden, Eisenblau, Ultramarine, Carmine etc. beschichtet sind und wobei die Farbe durch Variation der Schichtdicke bestimmt ist. Derartige Pigmente werden beispielsweise unter den Handelsbezeichnung Rona®, Colorona®, Dichrona® und Timiron® von der Firma Merck, Deutschland vertrieben. Organische Pigmente sind z.B. die natürlichen Pigmente Sepia, Gummigutt, Knochenkohle, Kasseler Braun, Indigo, Chlorophyll und andere Pflanzenpigmente. Synthetische organische Pigmente sind z.B. Azo-Pigmente, Anthrachinoide, Indigoide, Dioxazin-, Chinacridon-, Phtalocyanin-, Isoindolinon-, Perylen- und Perinon-, Metallkomplex-, Alkaliblau- und Diketopyrrolopyrrolpigmente.

### Treibmittel

Das Treibmittel ist in dem erfindungsgemäßen Aerosolhaarschaum vorzugsweise in einer Menge von 1 bis 20, besonders bevorzugt von 2 bis 10 Gew.%, bezogen auf die Summe aus verschäumbarer Zusammensetzung und Treibmittel, enthalten. Als Treibmittel sind beispielsweise niedere Alkane, z.B. n-Butan, i-Butan, Propan oder deren Gemische sowie Dimethylether oder Fluorkohlenwasserstoffe wie 1,1-Difluorethan oder Tetrafluorethan sowie ferner bei den in Betracht kommenden Drücken gasförmig vorliegende Treibmittel, z.B. N₂, N₂O und CO₂ sowie Gemische der vorstehend genannten Treibmittel geeignet. Besonders bevorzugt sind n-Butan, i-Butan, Propan und deren Gemische.

### Optionale Zusatzstoffe

Das erfindungsgemäße Produkt kann darüber hinaus die für Haarbehandlungsmittel üblichen Zusatzbestandteile enthalten, z.B. Parfümöle in einer Menge von 0,01 bis 1 Gew.%; Konservierungsmittel wie z.B. Parabene in einer Menge von 0,01 bis 1,0 Gew.%; Puffersubstanzen, wie z.B. Natriumcitrat oder Natriumphosphat, in einer Menge von 0,1 bis 1,0 Gew.%; Anfärbestoffe, wie z.B. Fluorescein Natriumsalz, in einer Menge von etwa 0,1 bis 1,0 Gew.%; Pflegestoffe, wie z.B. Pflanzen- und Kräuterextrakte, Protein- und Seidenhydrolysate, Lanolinderivate, in einer Menge von 0,1 bis 5 Gew.%; physiologisch verträgliche Silikonderivate, wie z.B. flüchtige oder nicht-flüchtige Silikonöle oder hochmolekulare Siloxanpolymere in einer Menge von 0,05 bis 20 Gew.%; Lichtschutzmittel, Antioxidantien, Radikalfänger, Antischuppenwirkstoffe, Fettalkohole, Vitamine, Weichmacher, rückfettende Agenzien, Glanzgeber und Kämmbarkeitsverbesserer in einer Menge von je etwa 0,01 bis 4 Gew.%.

### Verpackung

Das erfindungsgemäße Aerosolschaumprodukt wird in einer druckfesten Aerosolverpackung abgefüllt, welche mit einer Vorrichtung zum Verschäumen versehen wird. Als geeignete Schäumvorrichtung kann beispielsweise ein handelsüblicher Aersolschaumkopf verwendet werden. Beim Ausbringen aus diesr Aerosolverpackung bildet sich ein Schaum, welcher leicht in das Haar eingearbeitet werden kann und beim Einarbeiten auf dem Haar schnell zusammenbricht. Das Material der Verpackung kann aus Metall, Glas oder Kunststoff sein. Metalle sind z.B. Weißblech oder Aluminium. Im Falle von wasserhaltigen Formulierungen und Weißblech als Verpackungsmaterial können die üblichen Maßnahmen zur Vermeidung von Korrosionen getroffen werden, z.B. eine Beschichtung des Materials und/oder die Verwendung von Korrosionsinhibitoren. In einer bevorzugten Ausführungsform ist das Behältermaterial transparent oder durchsichtig, sodass die Konsistenz, die Füllstandshöhe, die Farbe der Zusammensetzung der flüssigen Phase von außen erkennbar ist. Geeignete transparente oder durchsichtige Materialien sind Glas und Kunststoffe wie z.B. Polyethylenterephtalat.

Eine besonders bevorzugte Ausführungsform ist ein Aerosolschaumprodukt zur Haarbehandlung, welches eine flüssige Zusammensetzung aufweist mit einem Gehalt an
(A) 0,5 bis 10 Gew.% mindestens eines Copolymers, ausgewählt aus Acrylates/Steareth-20 Itaconate Copolymeren und Acrylates/Ceteth-20 Itaconate Copolymeren,
(B) einer Base in einer Menge, die ausreichend ist, um das Copolymer (A) zu 50 bis 100% zu neutralisieren,
(C) 50-98 Gew.% eines Lösungsmittels, ausgewählt aus Wasser, Ethanol, n-Propanol, Isopropanol oder deren Gemisch,
(D) 0,5 bis 10 Gew.% eines zusätzlichen haarfestigenden Polymers,
(E) 0,01 bis 10 Gew.% eines Tensids
wobei die flüssige Zusammensetzung mindestens eine der folgenden rheologischen Eigenschaften aufweist:
- Viskosität von mindestens 50 mPa s bei 25°C und einer Schergeschwindigkeit von 50 s⁻¹;
- Auftreten eines Maximums in der Viskositätskurve bei ansteigender Schergeschwindigkeit;
- Viskositätsabnahme bei steigender Schergeschwindigkeit (shear thinning) oberhalb einer Schergeschwindigkeit von 20 s⁻¹;
- ein Elastizitätsmodul G' von mindestens 60 Pa bei 25°C, einer Messfrequenz von 1 Hz und einer Scherspannung von 2 Pa,
und wobei das Produkt ein Treibmittel in einer Menge von 1 bis 20 Gew.%, bezogen auf die Summe aus verschäumbarer Zusammensetzung und Treibmittel, enthält und das Treibmittel ausgewählt ist aus Propan, n-Butan, Isobutan und deren Gemischen.

### Anwendung

Das erfindungsgemäße kosmetische Mittel wird angewendet, indem es in einer zur Erzielung des gewünschten haarpflegenden oder haarfestigenden Effektes ausreichenden Menge auf vorzugsweise feuchtes, handtuchtrockenes Haar aufgetragen und eingearbeitet wird und ohne Ausspülen im Haar belassen wird. Anschließend kann die Frisur in üblicher Weise geformt werden bzw. können die Haare eingelegt und zum Schluss trocken gefönt werden. Es ist aber auch möglich, das Mittel direkt auf trockenem Haar anzuwenden.

Die nachfolgenden Beispiele sollen den Gegenstand der Erfindung näher erläutern. Die in den Beispielen angegebenen Polymergehalte beziehen sich jeweils auf den Feststoffgehalt.

### Beispiele

### Beispiel 1: Elastischer Aerosol-Schaumfestiger, 8% VOC

| | |
|---|---|
| 3 g | Structure® 3001¹⁾ |
| 0,5 g | Aminomethylpropanol (95%ig) |
| 0,2 g | Laureth-7 |
| 0,17 g | Parfüm |
| 0,15 g | Cetyltrimethylammoniumchlorid |
| ad 100 g | Wasser |

| | |
|---|---|
| ¹⁾ Acrylates/Ceteth-20 Itaconate Copolymer, 30%ig in Wasser | |

Die Wirkstoffmischung wird im Verhältnis 92:8 mit Propan/Butan 4.8 bar als Treibmittel in einem Aluminiumaerosolbehälter mit Schaumventil abgefüllt.

### Beispiel 2: Elastischer Aerosol-Schaumfestiger, 6% VOC

| | |
|---|---|
| 10 g | Vinylpyrrolidon/Vinylacetat Copolymer |
| 3 g | Structure® 3001¹⁾ |
| 0,5 g | Triethanolamin |
| 0,2 g | Oleth-5 |
| 0,17 g | Parfüm |
| 0,15 g | Cetyltrimethylammoniumbromid |
| ad 100 g | Wasser |

Die Wirkstoffmischung wird im Verhältnis 94:6 mit Propan/Butan 4.8 bar als Treibmittel in einem Aluminiumaerosolbehälter mit Schaumventil abgefüllt.

### Beispiel 3: Elastischer Aerosol-Schaumfestiger, 8% VOC

| | |
|---|---|
| 10 g | Polyvinylpyrrolidon K85 |
| 3 g | Structure® 2001²⁾ |
| 0,5 g | Tetrahydroxypropylethylendiamin |
| 0,2 g | Laureth-7 |
| 0,17 g | Parfüm |
| 0,15 g | Cetyltrimethylammoniumchlorid |
| ad 100 g | Wasser |

| | |
|---|---|
| ²⁾Acrylates/Steareth-20 Itaconate Copolymer, 30%ig in Wasser | |

Die Wirkstoffmischung wird im Verhältnis 92:8 mit Propan/Butan 2,7 bar als Treibmittel in einem Aerosolbehälter aus Glas oder Polyethylenterephtalat mit Schaumventil abgefüllt.

### Beispiel 4: Elastischer Aerosol-Schaumfestiger, 6% VOC

| | |
|---|---|
| 10 g | Polyvinylpyrrolidon K90 |
| 3 g | Structure® 3001¹⁾ |
| 0,5 g | Aminomethylpropanol (95%) |
| 0,2 g | Laureth-4 |
| 0,17 g | Parfüm |
| 0,2 g | PEG-12 Dimethicone |
| ad 100 g | Wasser |

Die Wirkstoffmischung wird im Verhältnis 94:6 mit Propan/Butan 4.8 bar als Treibmittel in einem Aluminiumaerosolbehälter mit Schaumventil abgefüllt.

### Beispiel 5: Elastischer Aerosol-Schaumfestiger, 8% VOC

| | |
|---|---|
| 10 g | Fructose |
| 3 g | Structure® 3001¹⁾ |
| 0,5 g | Aminomethylpropanol (95%) |
| 0,2 g | Laureth-4 |
| 0,17 g | Parfüm |
| 0,15 g | Cetrimoniumchlorid |
| ad 100 g | Wasser |

Die Wirkstoffmischung wird im Verhältnis 92:8 mit Propan/Butan 4.8 bar als Treibmittel in einem Aluminiumaerosolbehälter mit Schaumventil abgefüllt.

### Beispiel 6: Elastic Styler

| | |
|---|---|
| VA/Crotonates Copolymer (Luviset® 66) | 1,0 g |
| Acrylates/Ceteth-20 Itaconate Copolymer | 2,25 g |
| Cetrimoniumbromid | 0,15 g |
| Aminomethylpropanol | 1,4 g |
| PEG-40 Hydrogenated Castor Oil | 0,3 g |
| Parfüm | 0,2 g |
| Wasser | ad 100 g |

| | |
|---|---|
| Viskosität ca. 5800 mPa s (25°C) | |

Die Wirkstoffmischung wird im Verhältnis 92:8 mit Propan/Butan 4.8 bar als Treibmittel in einem Aluminiumaerosolbehälter mit Schaumventil abgefüllt.

### Beispiel 7: Elastic Styler mit Farbpigment

| | |
|---|---|
| VA/Crotonates Copolymer (Luviset® 66) | 1,0 g |
| Acrylates/Ceteth-20 Itaconate Copolymer | 2,25 g |
| Aminomethylpropanol | 1,4 g |
| Polysorbate 40 | 0,8 g |
| PEG-12 Dimethicone | 0,2 g |
| Timiron® Gold Plus MP-25 ³⁾ | 0,2 g |
| Wasser | ad 100 g |

| | |
|---|---|
| ³⁾ Mica/Titandioxid/Eisenoxid-Pigment mit rot-goldener Reflektionsfarbe Viskosität ca. 5600 mPa s (25°C) | |

Die Wirkstoffmischung wird im Verhältnis 92:8 mit Propan/Butan 4.8 bar als Treibmittel in einem Aluminiumaerosolbehälter mit Schaumventil abgefüllt.

### Beispiel 8: Elastic Styler

| | |
|---|---|
| Octylacrylamide/Acrylates/Butylaminoethyl Methacrylate Copolymer (Amphomer®) | 2,5 g |
| Acrylates/Ceteth-20 Itaconate Copolymer | 1,5 g |
| Aminomethylpropanol | 1,23 g |
| Cetrimoniumchlorid | 0,2 g |
| FD&C Green No. 3 (CI 42053) | 0,1 g |
| Wasser | ad 100 g |

| | |
|---|---|
| Viskosität ca. 3600 mPa s (25°C) | |

Die Wirkstoffmischung wird im Verhältnis 92:8 mit Propan/Butan 4.8 bar als Treibmittel in einem Aluminiumaerosolbehälter mit Schaumventil abgefüllt.

### Beispiel 9: Farb-Schaumfestiger

| | |
|---|---|
| Polyvinylpyrrolidon K80 | 2,0 g |
| Acrylates/Ceteth-20 Itaconate Copolymer | 3,75 g |
| Aminomethylpropanol | 2,0 g |
| Polysorbate 40 | 0,8 g |
| Cetrimoniumbromid | 0,17 g |
| 3-((2-Nitro-4-(trifluormethyl)phenyl)-amino)-1,2-propandiol | 0,11 g |
| Parfüm | 0,1 g |
| Wasser | ad 100 g |

| | |
|---|---|
| Viskosität ca. 14200 mPa s (25°C) | |

Die Wirkstoffmischung wird im Verhältnis 94:6 mit Propan/Butan 4.8 bar als Treibmittel in einem Aluminiumaerosolbehälter mit Schaumventil abgefüllt.

### Beispiel 10: Haar-Schaumgel

| | |
|---|---|
| PVP/VA Copolymer | 1,0 g |
| Acrylates/Ceteth-20 Itaconate Copolymer | 3,0 g |
| Aminomethylpropanol | 1,6 g |
| Wasser | ad 100 g |

| | |
|---|---|
| Viskosität ca. 6550 mPa s (25°C) | |

Die Wirkstoffmischung wird im Verhältnis 94:6 mit Propan/Butan 4.8 bar als Treibmittel in einem Aluminiumaerosolbehälter mit Schaumventil abgefüllt. In Figur 1 sind die Fließ- und Viskositätskurven der Wirkstoffmischung bei zuund abnehmender Schergeschwindigkeit im Bereich von 0 bis 20 s⁻¹ dargestellt.

## Patentansprüche

1. Aerosolschaumprodukt zur Haarbehandlung, umfassend eine verschäumbare Zusammensetzung mit einem Gehalt an
(A) mindestens einem Copolymeren, welches gebildet ist aus mindestens einer ersten radikalisch polymerisierbaren Monomerart, ausgewählt aus Itaconsäuremonoestern der allgemeinen Formel CH₂=C(COOR¹)CH₂COOR₂ wobei einer der Substituenten R¹ und R² für Wasserstoff und der andere für die Gruppe -(CH₂CH₂O)ₓ-R³ steht, x eine Zahl zwischen 1 und 100 bedeutet und R³ eine Alkylgruppe mit 8 bis 30 C-Atomen bedeutet und mindestens einer zweiten radikalisch polymerisierbaren Monomerart, ausgewählt aus Acrylat-Monomeren
(B) mindestens einer Base zur teilweisen oder vollständigen Neutralisation des Copolymers (A) und
(C) einem geeigneten Lösungsmittelsystem,
wobei die Zusammensetzung zusammen mit mindestens einem Treibmittel in einer druckfesten Verpackung abgefüllt ist und die Verpackung eine Vorrichtung zum Verschäumen aufweist.

2. Schaumprodukt nach Anspruch 1, **dadurch gekennzeichnet, dass** das Copolymer (A) in einer Menge von 0,1 bis 20 Gew.% bezogen auf die verschäumbare Zusammensetzung vorliegt.

3. Schaumprodukt nach einem der vorhergehenden Ansprüche, **dadurch gekennzeichnet, dass** bei der ersten Monomerart des Copolymers (A) R³ für eine Alkylgruppe mit 12 bis 20 C-Atomen und x für eine Zahl von 10 bis 40 steht und/oder dass die zweite Monomerart des Copolymers (A) ausgewählt ist aus Acrylsäure, Methacrylsäure, Acrylsäurealkylestern und Methacrylsäurealkylestern, wobei die Alkylgruppen 1 bis 10 C-Atome aufweisen.

4. Schaumprodukt nach einem der vorhergehenden Ansprüche, **dadurch gekennzeichnet, dass** das Copolymer (A) ausgewählt ist aus Acrylates/Steareth-20 Itaconate Copolymeren und Acrylates/Ceteth-20 Itaconate Copolymeren.

5. Schaumprodukt nach einem der vorhergehenden Ansprüche, **dadurch gekennzeichnet, dass** die aufgeschäumte Zusammensetzung soweit stabil ist, dass nach 30 min das Schaumvolumen mindestens 50% des Schaumvolumens unmittelbar nach der Ausbringung aus der Verpackung beträgt.

6. Schaumprodukt nach einem der vorhergehenden Ansprüche, **dadurch gekennzeichnet, dass** die Base (B) ausgewählt ist aus Aminoalkylalkoholen und/oder in einer Menge eingesetzt wird, sodass das Copolymer (A) zu 50 bis 100% neutralisiert ist.

7. Schaumprodukt nach einem der vorstehenden Ansprüche, **dadurch gekennzeichnet, dass** zusätzlich mindestens ein Tensid enthalten ist.

8. Schaumprodukt nach Anspruch 7, **dadurch gekennzeichnet, dass** das Tensid in einer Menge von 0,01 bis 15 Gew.%, bezogen auf die verschäumbare Zusammensetzung, eingesetzt wird und/oder ausgewählt ist aus
- nichtionischen Tensiden, ausgewählt aus Fettsäureglyceridethoxylaten, Fettalkoholethoxylaten, Fettaminethoxylaten, Fettsäurealkanolamidethoxylaten, Fettsäureesterethoxylaten, ethoxylierten Fettsäurezuckerestern, ethoxylierten Sorbitanfettsäureestern, nichtethoxylierten Fettsäurezuckerestern und Alkylpolyglycosiden und
- kationischen Tensiden der allgemeinen Formel (I)
N⁽⁺⁾R¹R²R³R⁴ X⁽⁻⁾ (I)
wobei R1 bis R4 unabhängig voneinander Alkylgruppen, Arylgruppen, Alkoxygruppen, Polyoxyalkylengruppen, Alkylamidogruppen, Hydroxyalkylgruppen oder Alkarylgruppen mit 1 bis 22 C-Atomen bedeuten und X⁽⁻⁾ ein Anion darstellt.

9. Schaumprodukt nach einem der vorstehenden Ansprüche, **dadurch gekennzeichnet, dass** das Treibmittel in einer Menge von 1 bis 20 Gew.%, bezogen auf die Summe aus verschäumbarer Zusammensetzung und Treibmittel, eingesetzt wird und/oder das Treibmittel ausgewählt ist aus Propan, i-Butan, n-Butan, Dimethylether, fluorierten Kohlenwasserstoffen oder aus Mischungen dieser Treibgase.

10. Schaumprodukt nach einem der vorstehenden Ansprüche, **dadurch gekennzeichnet, dass** es zusätzlich mindestens einen haarfestigenden Wirkstoff enthält.

11. Schaumprodukt nach Anspruch 10, **dadurch gekennzeichnet, dass** der haarfestigende Wirkstoff in einer Menge von 0,01 bis 15 Gew.% eingesetzt wird und/oder ausgewählt ist aus Sacchariden, Polyvinylpyrrolidon, Vinylpyrrolidon/Vinylacetat Copolymeren, Vinylacetat/Crotonsäure Copolymeren, Terpolymeren aus Vinylacetat, Crotonat und Vinylalkanoat, partialveresterten Copolymeren zwischen Vinylmethylether und Maleinsäureanhydrid und Copolymeren der Acrylsäure oder Methacrylsäure mit Monomeren ausgewählt aus Acrylsäure- oder Methacrylsäureestern, Acrylamiden, Methacrylamiden und Vinylpyrrolidon.

12. Schaumprodukt nach einem der vorstehenden Ansprüche, **dadurch gekennzeichnet, dass** es zusätzlich mindestens einen haarfärbenden Stoff enthält.

13. Schaumprodukt nach einem der vorstehenden Ansprüche, **dadurch gekennzeichnet, dass** die verschäumbare Zusammensetzung mindestens eine der folgenden rheologischen Eigenschaften aufweist:
- Viskosität von mindestens 50 mPa s bei 25°C und einer Schergeschwindigkeit von 50 s⁻¹;
- Auftreten eines Maximums in der Viskositätskurve bei ansteigender Schergeschwindigkeit;
- Viskositätsabnahme bei steigender Schergeschwindigkeit (shear thinning) oberhalb einer Schergeschwindigkeit von 20 s⁻¹;
- ein Elastizitätsmodul G' von mindestens 60 Pa, gemessen bei 25°C, einer Messfrequenz von 1 Hz und einer Scherspannung von 2 Pa.

14. Schaumprodukt nach Anspruch 13, **dadurch gekennzeichnet, dass** das Viskositätsmaximum bei steigender Schergeschwindigkeit im Bereich zwischen 0,1 und 5 s⁻¹ liegt.

15. Schaumprodukt nach einem der vorstehenden Ansprüche, **dadurch gekennzeichnet, dass** die druckfeste Verpackung aus einem transparenten oder durchsichtigen Material gefertigt ist.

16. Schaumprodukt nach einem der vorstehenden Ansprüche, **dadurch gekennzeichnet, dass** es eine flüssige Zusammensetzung aufweist mit einem Gehalt an
(A) 0,5 bis 10 Gew.% mindestens eines Copolymers, ausgewählt aus Acrylates/Steareth-20 Itaconate Copolymeren und Acrylates/Ceteth-20 Itaconate Copolymeren,
(B) einer Base in einer Menge, die ausreichend ist, um das Copolymer (A) zu 50 bis 100% zu neutralisieren,
(C) 50-98 Gew.% eines Lösungsmittels, ausgewählt aus Wasser, Ethanol, n-Propanol, Isopropanol oder deren Gemisch,
(D) 0,5 bis 10 Gew.% eines zusätzlichen haarfestigenden Polymers,
(E) 0,01 bis 10 Gew.% eines Tensids
wobei die flüssige Zusammensetzung mindestens eine der folgenden rheologischen Eigenschaften aufweist:
- Viskosität von mindestens 50 mPa s bei 25°C und einer Schergeschwindigkeit von 50 s⁻¹;
- Auftreten eines Maximums in der Viskositätskurve bei ansteigender Schergeschwindigkeit;
- Viskositätsabnahme bei steigender Schergeschwindigkeit (shear thinning) oberhalb einer Schergeschwindigkeit von 20 s⁻¹;
- ein Elastizitätsmodul G' von mindestens 60 Pa, gemessen bei 25°C, einer Messfrequenz von 1 Hz und einer Scherspannung von 2 Pa,
und wobei das Produkt ein Treibmittel in einer Menge von 1 bis 20 Gew.%, bezogen auf die Summe aus verschäumbarer Zusammensetzung und Treibmittel, enthält und das Treibmittel ausgewählt ist aus Propan, n-Butan, Isobutan und deren Gemischen.

## Claims

1. An aerosol foam product for treating hair, comprising a foamable composition having a content of
(A) at least one copolymer, which is formed from at least one first radically polymerizable kind of monomer, selected from itaconic acid monoesters having the general formula CH₂=C(COOR¹)CH₂COOR², wherein one of the substituents R¹ and R² stands for hydrogen and the other for the group -(CH₂CH₂O)ₓ-R³, x is a number between 1 and 100 and R³ is an alkyl group having 8 to 30 carbon atoms, and at least one second radically polymerizable kind of monomer selected from acrylate monomers
(B) at least one base for partially or completely neutralizing the copolymer (A), and
(C) a suitable solvent system,
wherein the composition is filled into a pressure-resistant packaging together with at least one propellant, and the packaging has a device for foaming.

2. The foam product according to Claim 1, **characterized in that** the copolymer (A) is available in a quantity of from 0.1 to 20% by weight relative to the foamable composition.

3. The foam product according to any one of the preceding claims, **characterized in that** in the first kind of monomer of the copolymer (A), R³ stands for an alkyl group having 12 to 20 carbon atoms and x for a number from 10 to 40, and/or that the second kind of monomer of the copolymer (A) is selected from acrylic acid, methacrylic acid, acrylic acid alkyl esters and methacrylic acid alkyl esters, the alkyl group having 1 to 10 carbon atoms.

4. The foam product according to any one of the preceding claims, **characterized in that** the copolymer (A) is selected from acrylates/steareth-20 itaconate copolymers and acrylates/ceteth-20 itaconate copolymers.

5. The foam product according to any one of the preceding claims, **characterized in that** the foamed composition is so stable that the foam volume after 30 minutes is at least 50% of the foam volume immediately after the initial release from the packaging.

6. The foam product according to any one of the preceding claims, **characterized in that** the base (B) is selected from amino alkyl alcohols and/or is used in a quantity such that 50 to 100% of the copolymer (A) are being neutralized.

7. The foam product according to any one of the preceding claims, **characterized in that** it additionally contains at least one surfactant.

8. The foam product according to Claim 7, **characterized in that** the surfactant is used in a quantity of from 0.01 to 15% by weight, relative to the foamable composition, and/or is selected from
- nonionic surfactants, selected from fatty acid glycerol ethoxylates, fatty alcohol ethoxylates, fatty amine ethoxylates, fatty acid alkanolamide ethoxylates, fatty acid ester ethoxylates, ethoxylated fatty acid sugar esters, ethoxylated sorbitan fatty acid esters, non-ethoxylated fatty acid sugar esters and alkyl polyglycosides, and
- cationic surfactants having the general formula (I)
n⁽⁺⁾R¹ R²R³R⁴ x⁽⁻⁾ (I)
wherein R1 to R4, independently of one another, are alkyl groups, aryl groups, alkoxy groups, polyoxy alkylene groups, alkylamido groups, hydroxyalkyl groups or alkaryl groups having 1 to 22 carbon atoms, and X⁽⁻⁾ represents an anion.

9. The foam product according to any one of the preceding claims, **characterized in that** the propellant is used in a quantity of from 1 to 20% by weight, relative to the sum of foamable composition and propellant, and/or the propellant is selected from propane, i-butane, n-butane, dimethylether, fluorinated hydrocarbons or mixtures of these propellants.

10. The foam product according to any one of the preceding claims, **characterized in that** it additionally contains at least one hair setting active agent.

11. The foam product according to Claim 10, **characterized in that** the hair setting active agent is used in a quantity of from 0.01 to 15% by weight and/or is selected from saccharides, polyvinyl pyrrolidone, vinyl pyrrolidone/vinyl acetate copolymers, vinyl acetate/crotonic acid copolymers, terpolymers of vinyl acetate, crotonate and vinyl alkanoate, partially esterified copolymers between vinyl methyl ether and maleic acid anhydride, and copolymers of acrylic acid or methacrylic acid with monomers selected from acrylic acid esters or methacrylic acid esters, acrylamides, methacrylamides and vinyl pyrrolidone.

12. The foam product according to any one of the preceding claims, **characterized in that** it additionally contains at least one hair dyeing substance.

13. The foam product according to any one of the preceding claims, **characterized in that** the foamable composition has at least one of the following rheological properties:
- viscosity of at least 50 mPa·s at 25 °C and a shear rate of 50 s⁻¹;
- occurrence of a maximum in the viscosity curve with increasing shear rate;
- viscosity decrease with increasing shear rate (shear thinning) above a shear rate of 20 s⁻¹ ;
- a modulus of elasticity G' of at least 60 Pa, as measured at 25°C, measuring frequency of 1 Hz, and shear stress of 2 Pa.

14. The *foam product* according to Claim 13, **characterized in that**, with increasing shear rate, the viscosity maximum lies in the range between 0.1 and 5 s⁻¹.

15. The foam product according to any one of the preceding claims, **characterized in that** the pressure-resistant packaging is made from a transparent or see-through material.

16. The foam product according to any one of the preceding claims, **characterized in that** it has a liquid composition having a content of
(A) 0.5 to 10% by weight of at least one copolymer, selected from acrylates/steareth-20 itaconate copolymers and acrylates/ceteth-20 itaconate copolymers,
(B) a base in a quantity sufficient to neutralize 50 to 100% of the copolymer (A),
(C) 50-98% by weight of a solvent, selected from water, ethanol, n-propanol, isopropanol, or mixtures thereof,
(D) 0.5 to 10% by weight of an additional hair setting polymer,
(E) 0.01 to 10% by weight of a surfactant,
wherein the liquid composition has at least one of the following rheological properties:
- viscosity of at least 50 mPa·s at 25 °C and a shear rate of 50 s⁻¹;
- occurrence of a maximum in the viscosity curve with increasing shear rate;
- viscosity decrease with increasing shear rate (shear thinning) above a shear rate of 20 s⁻¹;
- a modulus of elasticity G' of at least 60 Pa, measured at 25 °C, measuring frequency of 1 Hz, and shear stress of 2 Pa,
and wherein the product contains the propellant in a quantity of from 1 to 20% by weight, relative to the sum of foamable composition and propellant, and the propellant is selected from propane, n-butane, isobutane, and mixtures thereof.

## Revendications

1. Produit de mousse en aérosol pour le traitement des cheveux, comprenant une composition moussable ayant un contenu de
(A) au moins un copolymère, qui est formé d'au moins un premier type de monomère polymérisable par radical, choisi parmi les monoesters d'acide itaconique de formule générale CH₂=C(COOR¹)CH₂COOR², dans laquelle un des substituants R¹ et R² représente l'hydrogène et l'autre pour le groupe - (CH₂CH₂O)ₓ-R³, x est un nombre compris entre 1 et 100 et R³ est un groupe alkyle comportant 8 à 30 atomes de carbone, et au moins un deuxième type de monomère polymérisable par radical choisi parmi des monomères acrylate
(B) au moins une base pour neutraliser partiellement ou complètement le copolymère (A), et
(C un système de solvant approprié,
où la composition est remplie dans un conditionnement résistant à la pression conjointement avec au moins un propulseur, et le conditionnement a un dispositif pour le moussage.

2. Produit de mousse selon la revendication 1, **caractérisé en ce que** le copolymère (A) est disponible en une quantité allant de 0,1 à 20 % en poids par rapport à la composition moussable.

3. Produit de mousse selon l'une quelconque des revendications précédentes, **caractérisé en ce que** le premier type de monomère du copolymère (A), R³ représente un groupe alkyle comportant 12 à 20 atomes de carbone et x un nombre de 10 à 40, et/ou **en ce que** le deuxième type de monomère du copolymère (A) est choisi parmi l'acide acrylique, l'acide méthacrylique, des alkylesters d'acide acrylique et des alkylesters d'acide méthacrylique, le groupe alkyle comportant 1 à 10 atomes de carbone.

4. Produit de mousse selon l'une quelconque des revendications précédentes, **caractérisé en ce que** le copolymère (A) est choisi parmi des copolymères acrylates/stéareth-20 itaconate et des copolymères acrylates/céteth-20 itaconate.

5. Produit de mousse selon l'une quelconque des revendications précédentes, **caractérisé en ce que** la composition de mousse est si stable que le volume de mousse après 30 minutes est au moins 50 % du volume de mousse immédiatement après la libération initiale à partir du conditionnement.

6. Produit de mousse selon l'une quelconque des revendications précédentes, **caractérisé en ce que** la base (B) est choisie parmi des alcools amino alkyliques et/ou est utilisée en une quantité telle que 50 à 100 % du copolymère (A) sont neutralisés.

7. Produit de mousse selon l'une quelconque des revendications précédentes, **caractérisé en ce qu'**il contient en outre au moins un agent tensioactif.

8. Produit de mousse selon la revendication 7, **caractérisé en ce que** l'agent tensioactif est utilisé en une quantité allant de 0,01 à 15 % en poids, par rapport à la composition moussable, et/ou est choisi parmi
- des agents tensioactifs non ioniques, choisis parmi les éthoxylates d'acide gras de glycérol, les éthoxylates d'alcool gras, les éthoxylates d'amine grasse, les éthoxylates d'alcanolamide d'acide gras, les éthoxylates d'ester d'acide gras, les esters de sucre d'acide gras éthoxylés, les esters d'acide gras de sorbitan éthoxylés, les esters de sucre d'acide gras non éthoxylés et les alkyl-polyglycosides, et
- des agents tensioactifs cationiques de formule générale (I)
n⁽⁺⁾R¹R²R³R⁴ x⁽⁻⁾ (I)
dans laquelle R1 à R4, indépendamment l'un de l'autre, sont des groupes alkyle, des groupes aryle, des groupes alcoxy, des groupes polyoxy alkylène, des groupes alkylamido, des groupes hydroxyalkyle ou des groupes alkaryle ayant 1 à 22 atomes de carbone, et X⁽⁻⁾ représente un anion.

9. Produit de mousse selon l'une quelconque des revendications précédentes, **caractérisé en ce que** le propulseur est utilisé en une quantité allant de 1 à 20 % en poids, par rapport à la somme de la composition moussable et du propulseur, et/ou le propulseur est choisi parmi le propane, l'i-butane, le n-butane, l'éther diméthylique, des hydrocarbures fluorés ou des mélanges de ces propulseurs.

10. Produit de mousse selon l'une quelconque des revendications précédentes, **caractérisé en ce qu'**il contient en outre au moins un agent actif de fixation des cheveux.

11. Produit de mousse selon la revendication 10, **caractérisé en ce que** l'agent actif de fixation des cheveux est utilisé en une quantité allant de 0,01 à 15 % en poids et/ou est choisi parmi les saccharides, le polyvinylpyrrolidone, des copolymères vinyl-pyrrolidone/acétate vinylique, des copolymères acétate vinylique/acide crotonique, des terpolymère acétate vinylique, crotonate et alcanoate vinylique, des copolymères partiellement estérifiés entre le vinyl méthyl éther et l'anhydride d'acide maléique, et des copolymères d'acide acrylique ou d'acide méthacrylique avec des monomères choisis parmi des esters d'acide acrylique ou des esters d'acide méthacrylique, des acrylamides, des méthacrylamides et le vinyl-pyrrolidone.

12. Produit de mousse selon l'une quelconque des revendications précédentes, **caractérisé en ce qu'**il contient, en outre, au moins une substance de teinture capillaire.

13. Produit de mousse selon l'une quelconque des revendications précédentes, **caractérise en ce que** la composition moussable a au moins une des propriétés rhéologiques suivantes :
- une viscosité d'au moins 50 MPa·s à 25 °C et une vitesse de cisaillement de 50 s⁻¹ ;
- la présence d'un maximum dans la courbe de viscosité avec une vitesse de cisaillement croissante ;
- une diminution de viscosité avec une vitesse de cisaillement croissante (rhéofluidification) au-dessus d'une vitesse de cisaillement de 20 s⁻¹;
- un module d'élasticité G' d'au moins 60 Pa, tel que mesuré à 25 °C, en mesurant une fréquence de 1 Hz, et une contrainte de cisaillement de 2 Pa.

14. Produit de mousse selon la revendication 13, **caractérisé en ce que**, avec une vitesse de cisaillement croissante, le maximum de viscosité se trouve dans la gamme comprise entre 0,1 et 5 s⁻¹.

15. Produit de mousse selon l'une quelconque des revendications précédentes, **caractérisé en ce que** le conditionnement résistant à la pression est fabriqué dans un matériau transparent ou translucide.

16. Produit de mousse selon l'une quelconque des revendications précédentes, **caractérisé en ce qu'**il a une composition liquide ayant un contenu de
(A) 0,5 à 10 % en poids d'au moins un copolymère, choisi parmi les copolymères acrylates/stéareth-20 itaconate et les copolymères acrylates/céteth-20 itaconate,
(B) une base en une quantité suffisante pour neutraliser 50 à 100 % du copolymère (A),
(C) 50 à 98 % en poids d'un solvant, choisi parmi l'eau, l'éthanol, le n-propanol, l'isopropanol, ou leurs mélanges,
(D) 0,5 à 10 % en poids d'un polymère de fixation des cheveux supplémentaire,
(E) 0,01 à 10 % en poids d'un agent tensioactif,
où la composition liquide a au moins une des propriétés rhéologiques suivantes :
- une viscosité d'au moins 50 MPa·s à 25 °C et une vitesse de cisaillement de 50 s⁻¹;
- la présence d'un maximum dans la courbe de viscosité avec une vitesse de cisaillement croissante ;
- une diminution de viscosité avec une vitesse de cisaillement croissante (rhéofluidification) au-dessus d'une vitesse de cisaillement de 20 s⁻¹;
- un module d'élasticité G' d'au moins 60 Pa, mesuré à 25 °C, en mesurant une fréquence de 1 Hz, et une contrainte de cisaillement de 2 Pa.
et où le produit contient le propulseur en une quantité allant de 1 à 20 % en poids, par rapport à la somme de composition moussable et de propulseur, et le propulseur est choisi parmi le propane, le n-butane, l'isobutane, et leurs mélanges.
